# EUROPEAN PATENT SPECIFICATION

(11) **EP 2 606 859 B1**
(45) Date of publication and mention of the grant of the patent: **25.11.2015**
(21) Application number: 11382400.7
(22) Date of filing: 23.12.2011
(51) Int. Cl.: A61F 2/44

(54) **Osseointegrable implant for cervical corpectomy**
Osseointegrierbares Implantat für cervicale Korpektomie
Implant osseo-intégrable pour corpectomie des cervicales

(43) Date of publication of application: 26.06.2013
(73) Proprietor: LORTEK S. COOP., 20240 Ordizia (Gipuzkoa) (ES)
(72) Inventor: Echeverria Zubiria, Alberto, 20240 Ordizia (Gipuzkoa) (ES)
(74) Representative: Carpintero Lopez, Francisco

(56) References cited:
- EP-A2- 1 683 593
- EP-A2- 1 800 627
- WO-A1-2010/080511
- WO-A2-2008/022206
- DE-A1- 19 519 101
- US-B1- 6 206 924

## Description

### Technical Field of the Invention

The present invention is encompassed in the technical field of medical devices, and particularly in the sector of osseointegrable implants for cervical corpectomy.

### Background of the Invention

Applying prostheses in the spinal column is a technique which has been performed for many years. In the first half of the 20^{th} century, spinal fusion and the instruments thereof were developed and applied as techniques for treating spinal instability before the biomechanical principles involving spinal instability were understood.

In recent years, with the development of new manufacturing materials and processes, in addition to a more extensive study and a greater understanding of the biomechanical principles, vertebral replacement devices have evolved tremendously, becoming components which are indispensable in 21^{st} century neurosurgery treatments. The need for an additional surgical intervention in a patient for removing cortical bone, which is necessary in older applications, is thus prevented. It is estimated that approximately 400,000 vertebral replacements due to vertebral fractures alone were performed in the US in 2009, generating a worldwide income of more than 1 billion dollars. Furthermore, a clear trend towards an increased use of fusion surgery can be seen due to the success cases and good results obtained.

The spine is naturally stable as a result of its anatomical configuration and of the soft structures joining one vertebral body with the next. Nevertheless, if the spine is subjected to a trauma which goes beyond the natural resistance of the bone and ligament structures or if one develops a disease, a fracture or lesion of the parts occurs, causing the instability thereof.

Spinal instability is not only a condition but a pathological consequence of a variety of different spinal conditions, such as traumatic fractures, tumors, degenerative diseases and infections.

These disorders are resolved with different types of surgical approaches, but the surgical approaches which are most widely used today are the combination of corpectomy and posterior fixation.

The surgery necessary for inserting the implant for corpectomy is performed by anterior corpectomy and is done by means of an operation through the front skin folds in the neck. Depending on each case, the intervertebral discs, the vertebral body (part or all of it) are removed, and the area compressing the cord is liberated. Then, the implant may or may not be inserted by means of using minimally invasive surgery (MIS). Specific instruments will be necessary for said insertion. Subsequently, to prevent excessive movement between two vertebrae, the option of fixing the vertebrae with titanium plates is available.

The patient will normally need a postoperative cervical collar. After 4 or 5 months, the bone mass is adequately formed, thus achieving adequate implant fixation and the necessary spinal column stability.

An implant for corpectomy is a device the function of which is to replace a damaged or collapsed vertebral body. The replacement device is supported on the adjacent vertebrae, eliminating the movement between them and stabilizing the spine.

A system of plates screwed on the posterior face of the two adjacent vertebral bodies which will be in permanent contact with the implant for corpectomy may be necessary to achieve correct spinal stability and primary implant fixation. If said system is not implanted, outer instruments would be necessary until sufficient osseointegration (secondary fixation) is achieved and the implant is fixed. Since cement, screws or similar components are not used to fuse the implant and the adjacent vertebral bodies, and to thus stabilize the spine, achieving adequate osseointegration is essential. Due to this requirement, many vertebral body replacement device designs contain an inner cavity with holes in the walls which enable grafting in ground bone or synthetic materials facilitating said osseointegration. Therefore, the bone is inserted in the implant by using the holes of the wall, fusing the adjacent vertebrae and the implant for corpectomy. These grafts provide the scaffold or foundation so that new bone grows in the body of the patent and stimulate the production and fusion thereof. Surgeons have historically obtained healthy material for bone graft from the body of the patient himself/herself, generally from the iliac crest. Procuring the bone graft in this manner requires an additional surgery causing postoperative pain, and there is also a limit on the amount of bone which can be removed from the donating area. Therefore, the materials which are most widely used today for fulfilling said function are autografts (autogenous bone), xenografts and allografts, having their advantages and drawbacks. The fusion achieved between the adjacent vertebrae limits the movement of the members with respect to one another, whereby the implant is consolidated against axial stress, rotational stress and lateral displacements in the spine.

Another prominent point of the implant design is the possibility of adjusting the length thereof. Therefore, some designs offer the opportunity of adjusting the length of the implant *in situ* to the space between the adjacent vertebrae of each patient, providing the surgeon with a margin for improvisation in order to better respond to the specifications of each patient. Expandable implants of this type further provide significant clinical advantages for the patient, as well as a smaller incision due to the fact that the implant is narrower at the time of insertion since it expands inside the body of the patient.

The vertebral replacement device can be manufactured from any biocompatible material. The spinal column industry has traditionally been limited to manufacturing implants for corpectomy from titanium alloys, Co-Cr alloys, stainless steel, ceramic and combinations thereof mainly due to the fact that these materials have a good combination of mechanical and biocompatibility properties. Nevertheless, with the development of new materials and greater knowledge in handling them, their range has diversified. The incorporation on the market of synthetic or natural tissues which may or may not be absorbable can be understood in this context. One of these very widespread new materials is PEEK, mainly due to its radiolucency which allows the surgeons to view the state of fusion.

Known implants for corpectomy are described, for example, in documents US-4932975, US-6190413, US-6344057, US-6352556, US-6375683, US-6562074, US-6783547, US-6808537, US-6821298, US-6908485, US-6991653, US-7008453, US-7156874, US-7311733, US-7384431, US-7674296, US-7758648, US-7815683, US-7825134, US-7879096, US-2007/0068862-A1, US-2007/0250171-A1, US-2008/013060-A1, US-2008/0167720-A1, US 2009/0112325, US-2009/0138083-A1, US-2009/0138089-A1, US-2009/0164019-A1, US-2010/0028061-A1, US-2010249934-A1, DE-102008010607-A1, WO-2009/058578 and WO-2010/080511.

Nevertheless, conventional implants for corpectomy of the state of the art have structures which do not allow their quick and at the same time effective osseointegration since they have structures which do not offer effective support for the growth of the bone of the patient from the grafts introduced into the implant.

### Description of the Invention

The object of the present invention is to overcome the aforementioned drawbacks of the state of the art by means of an osseointegrable implant for cervical corpectomy intended for replacing a damaged vertebral body between two vertebral bodies adjacent to the damaged vertebral body comprising a cylindrical spacing body with an upper end and a lower end, a plurality of side holes communicated with the inside of the spacing body for introducing graft material, upper anchoring means protruding from the upper end of the spacing body for anchoring the implant to an upper adjacent vertebral body, as well as lower anchoring means protruding from the lower end of the spacing body for anchoring the implant to a lower adjacent vertebral body, in which
the spacing body comprises a reticular supporting structure comprising a plurality of laterally interconnected columns of three-dimensional unit cells attached to one another;
each unit cell encloses an inner space defined vertically between an upper base and a lower base of the unit cell;
the inner spaces of the unit cells are laterally demarcated between vertical connecting elements connecting the respective upper and lower bases of the cells,
whereby the columns of unit cells of the reticular structure are formed by a plurality of vertical bars attached to one another by means of braces extending between neighboring vertical bars;
the inner space of at least some of the unit cells is laterally demarcated between respective vertical sections of at least three neighboring vertical bars;
each vertical section is defined between the upper base and the lower base of the unit cell, and each brace of a unit cell connecting neighboring vertical bars extends between the upper base and the lower base of the unit cell such that the unit cells form stacked strata of unit cells.

The implant according to the present invention is obviously made of a biocompatible material, such as titanium or CoCr.

The reticular structure of the spacing body is based on the reticular structures (scaffolds) which are known for other applications in which supports are necessary in new bone formation and growth. According to the present invention, the reticular structure of the implants is intended to act as a support or scaffold facilitating new bone formation and growth, thus facilitating osseointegration (secondary fixation) of the implant, which must complement the fixation of the implant provided by the anchoring means (primary stability). Likewise, the reticular structure of the spacing body facilitates load distribution in the implant.

The vertical bars act in the direction of the compression loads and therefore provide rigidity to the implant.

The neighboring vertical bars are preferably arranged equidistantly from one another, whereas the braces connecting them to one another can extend horizontally or in an inclined manner between neighboring vertical bars. The braces in turn preferably have an inclination of 45°± 5° with respect to the vertical bars to which they are attached.

To optimize the mechanical biocompatibility, the side of the spacing body facing the spinal cord can be provided with at least one concave wall, for example in the form of a plate with a smooth outer surface, i.e., a curved wall intended to prevent from damaging the spinal cord.

In a preferred embodiment of the implant according to the invention, the spacing body comprises an upper body and a lower body coupled to one another by a vertical expansion mechanism for adjusting the implant in height between the two adjacent vertebral bodies. This expansion mechanism in turn comprises a screw which is threaded in respective fixed housings in the upper body and in the lower body and has an upper helical thread and a lower helical thread having opposite helical trajectories and a fixed outer flange located between said threads.

In order to facilitate applying the rotating movement, the outer flange can be provided with radial threaded passages communicated with the inner axial passage in which radial threaded passages the tip of a suitable instrument can be introduced. These threaded passages also serve for screwing a set-screw in at least one of the passages until it presses against the wall of the upper tube and therefore blocks the rotation of the screw.

The screw can have an inner axial passage through which it is guided in a sliding manner on a telescopic assembly formed by an upper cylinder fixed to the fixed housing of the upper body and a lower cylinder fixed to the fixed housing of the lower body which extend in the inner axial passage of the screw. One of the cylinders is a tube susceptible to sliding vertically on the other cylinder.

The tubes can in turn be provided with an anti-rotation system, such as for example a key in one of the cylinders and a tube-shaped keyway in the other tube. The key can be an outer axial rib in one of the tubes and in which case the keyway in the other tube is a recess complementary to said rib. Since the tube is vertically slidable on the lower cylinder, the telescopic assembly formed by the keyway and the key make the rotation between both cylinders impossible. On the other hand, since the fixed cylinders are located in the upper body and the lower body -1d-, respectively, the relative rotation between said bodies is also made impossible.

In a preferred embodiment of the invention, the braces extend in an inclined manner such that in at least some of the unit cells, each vertical bar is attached to at least another neighboring vertical bar by means of a pair of inclined braces with respective converging ends in the vertical section of a vertical bar in the intermediate part of the cell and respective diverging ends attached to the other neighboring bar in said upper part and in said lower part of the unit cell, respectively. According to this preferred embodiment, in each unit cell of this type, the diverging ends of each pair of braces are attached to a first vertical bar which is in turn attached to the diverging ends of at least two pairs of braces the converging ends of which are respectively attached to at least two second neighboring vertical bars that are close to one another. These second vertical bars are arranged radially with respect to the first vertical bar, and the braces attaching the second vertical bars with the first vertical bar have respective longitudinal axes intersecting with the first vertical bar, defining respective equiangular arches with respect to one another. Therefore, the inner space of each cell is defined between the first vertical bar and the second vertical bars.

The longitudinal axes of every two neighboring braces preferably define equiangular arches of 15° to 90° with respect to one another, and more preferably equiangular arches of 60°.

Since the function of the implant is to replace a damaged cervical vertebral body between two adjacent vertebral bodies, its height must be oriented in the dimension of the replaced vertebral body, i.e., about 18 mm, whereas its diameter conveniently must not exceed 16 to 17 mm.

The reticular structure of the spacing body is susceptible to offering porosity, i.e., an inner space, to allow housing the maximum amount of graft possible while it must at the same time satisfactorily resist all the stresses to which the implant is subjected such that the vertical bars and the braces must, on one hand, provide adequate rigidity to the implant and on the other be sized so as to not excessively reduce the porosity of the spacing body.

In this sense, in a convenient embodiment of the unit cells making up the spacing body, at least some of the unit cells can have a height of approximately 3 mm to 6 mm that allows it to have the height equivalent to that of a cervical vertebral body, approximately 18 mm, by means of superposing unit cell strata of the same height. In the case of an implant without an expansion mechanism, the spacing body can be formed from 6 to 3 strata of superposed unit cells from which there would be eliminated, in the case of an implant with an expansion mechanism, the strata of unit cells the place of which is being occupied by the part of the expansion mechanism occupying the place between the upper body and the lower body of the spacing body. The vertical bars and braces are in turn preferably cylindrical with a diameter which can be about 0.4 mm.

It must also be taken into account that before osseointegration and plasticization, the spacing body is the element of the implant which withstands the greatest loads, therefore it is desirable for its rigidity to be greater than that of the bone (having an apparent elastic modulus of about 15 GPa), while at the same time the rigidity is not too excessive to thus enable preventing negatively affecting the working of the bone and subsequent bone loss by incorporating implants with a rigidity different from the bone (stress shielding). Porosities of about 80% and adequate rigidities, such as for example 19-20 GPa of apparent elastic modulus, can advantageously be achieved with the design of the reticular structure.

The implant according to the present invention further offers the advantage that its reticular body, in view of the repetition of its unit cells, is susceptible to being manufactured preferably by additive manufacturing processes such as SLM (Selective Laser Melting) or EBM (Electron Beam Melting).

As inferred from the foregoing, the present invention effectively overcomes the drawbacks of the state of the art with a simple structure.

### Brief Description of the Drawings

Aspects and embodiments of the invention are described below based on several schematic drawings in which
Figure 1 is a front perspective view of an embodiment of an implant according to a first embodiment of the invention;
Figure 2 is a sectional view through line A-A marked in Figure 1;
Figure 3 is a front perspective view of an embodiment of an implant according to a second embodiment of the invention;
Figure 4 is a rear perspective view of the embodiment of the implant shown in Figure 3;
Figure 5 is a front perspective view showing the components of the implant shown in Figure 3;
Figure 6 is a cross-section view of the screw of the expansion mechanism of the implant shown in Figure 3;
Figure 7 is a side perspective view of a set-screw forming part of the expansion mechanism of the implant shown in Figure 3;
Figure 8 is a front perspective view of the upper body of the implant shown in Figure 3;
Figure 9 is a bottom perspective view of the rear part of the upper body of the implant shown in Figure 3;
Figure 10 is a front perspective view of the lower body of the implant shown in Figure 3;
Figure 11 is a top perspective view of the upper body of the implant shown in Figure 3;
Figure 12 is a top perspective view of a first embodiment of two adjacent unit cells corresponding to the horizontal strata of the implant according to the present invention;
Figure 13 is a top plan view of the unit cells shown in Figure 12;
Figure 14 shows top plan sectional views through line B-B of three columns formed by three strata of unit cells;
Figure 15 is a top plan view of the arrangement of the vertical bars and braces of a unit cell according to the first embodiment illustrated in Figure 12;
Figure 16 is a top plan view of the arrangement of the vertical bars and braces of a unit cell according to a second embodiment;
Figure 17 is a top plan view of the arrangement of the vertical bars and braces of a unit cell according to a third embodiment;
Figure 18 is a bottom plan view of an embodiment of the upper body of the implant with unit cells corresponding to Figures 15, 16 and 17;
Figure 19 is a view of a top perspective view of a second embodiment of the unit cell according to the present invention;
Figure 20 is a view of a top perspective view of a third embodiment of the unit cell according to the present invention;
Figure 21 is a view of a top perspective view of a fourth embodiment of the unit cell according to the present invention;
Figure 22 is a view of a top perspective view of a fifth embodiment of the unit cell according to the present invention;
Figure 23 is a view of a top perspective view of a sixth embodiment of the unit cell according to the present invention;
Figure 24 is a view of a top perspective view of a seventh embodiment of the unit cell according to the present invention;
Figure 25 is a view of a top perspective view of an eight embodiment of the unit cell according to the present invention.

Reference numbers identifying the following elements can be seen in these drawings:
- 1: spacing body
- 1a: upper end
- 1b: lower end
- 1c: upper body
- 1d: lower body
- 2: vertical bars
- 2': vertical sections
- 2a: first vertical bars
- 2b: second vertical bars
- 2c: vertical connecting walls
- 2d: angled vertical connecting bars
- 2e: through holes
- 3: unit cells
- 3a: upper base of the unit cell
- 3b: lower base of the unit cell
- 4: braces
- 5: side holes
- 6a: upper anchoring means
- 6b: lower anchoring means
- 7: screw
- 7a: upper helical thread
- 7b: lower helical thread
- 7c: outer flange
- 7d: inner axial passage
- 7e: radial threaded passages
- 7f: set-screw
- 7g: outer thread
- 7h: slot
- 8a, 8b: fixed housings
- 9a: upper tube
- 9b: lower cylinder
- 9c: keyway
- 9d: key
- 10: concave wall
- 11: inner space

### Embodiments of the Invention

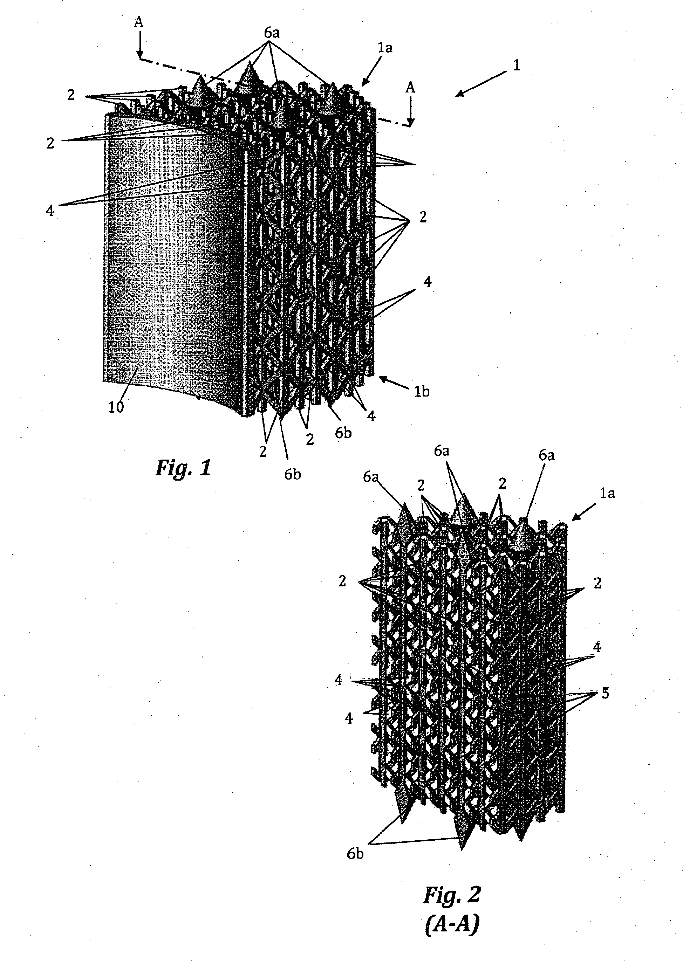

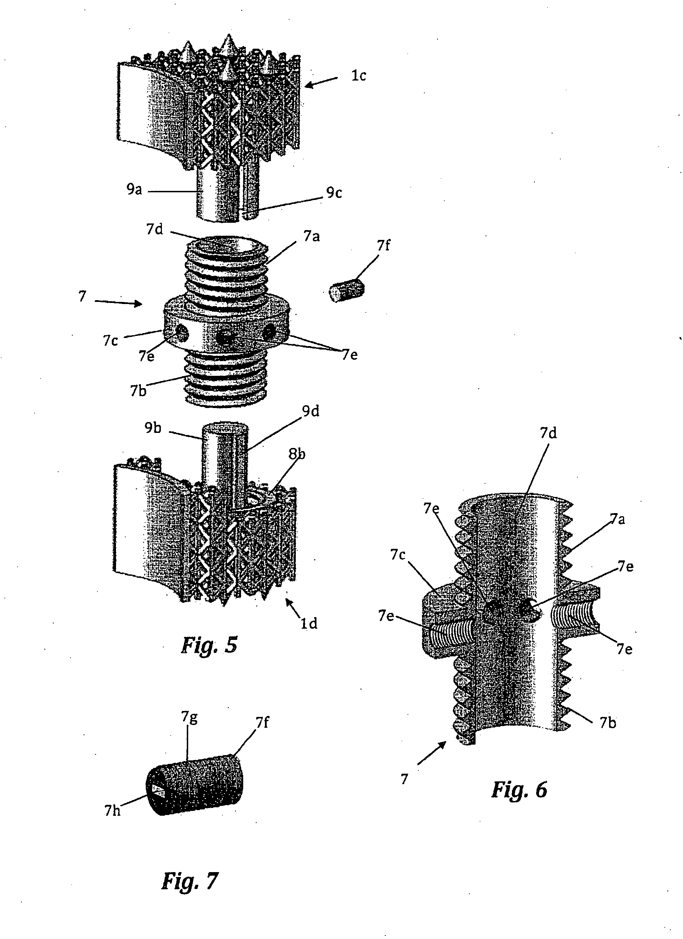

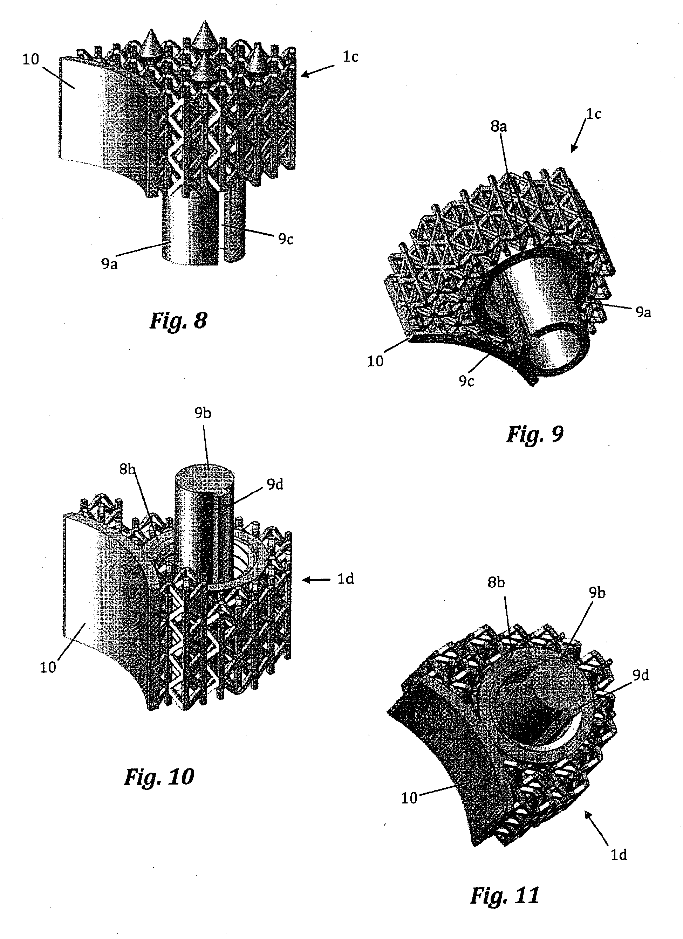

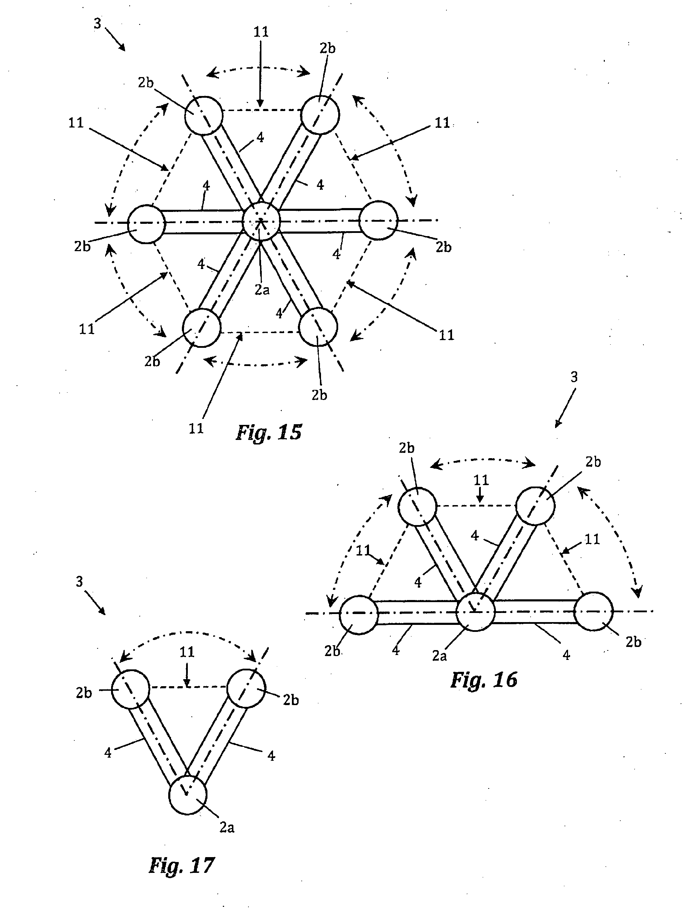

According to the embodiments shown in Figures 1 to 18, the osseointegrable implant for cervical corpectomy comprises a cylindrical spacing body -1- with an upper end -1a- and a lower end -1b-, and a reticular supporting structure comprising a plurality of columns of three-dimensional unit cells -3-. The columns -2- of unit cells -3- of the reticular structure are formed by a plurality of vertical bars -2- attached to one another by means of inclined braces -4- extending between neighboring vertical bars -2-. The reticular structure acts as a support or scaffold in new bone formation and growth thus facilitating implant osseointegration (secondary fixation).

The spacing body -1- further comprises a plurality of side holes -5- as well as upper anchoring means -6a- in the form of spikes protruding from the upper end -1a-of the spacing body -1- for anchoring the implant to an upper adjacent vertebral body (not shown in the drawings) and lower anchoring means -6b-, also in the form of spikes, protruding from the lower end -1 b- of the spacing body -1- for anchoring the implant to a lower adjacent vertebral body (not shown in the drawings). The spikes having a projecting height of 2 mm serve for the primary fixation of the implant in the adjacent vertebral bodies and since they are elements which occupy little space at the ends of the spacing body, they allow the bone of the adjacent vertebral bodies to grow through most of the surfaces of said ends, thus enhancing implant osseointegration.

In the embodiment illustrated in Figures 1 and 2, the spacing body -1- is formed by a single block of reticular structure and does not comprise any vertical expansion mechanism which allows adjusting the height of the implant *in situ.* This embodiment can be useful in specific cervical corpectomy applications, for example applications in which complete implant osseointegration is sought.

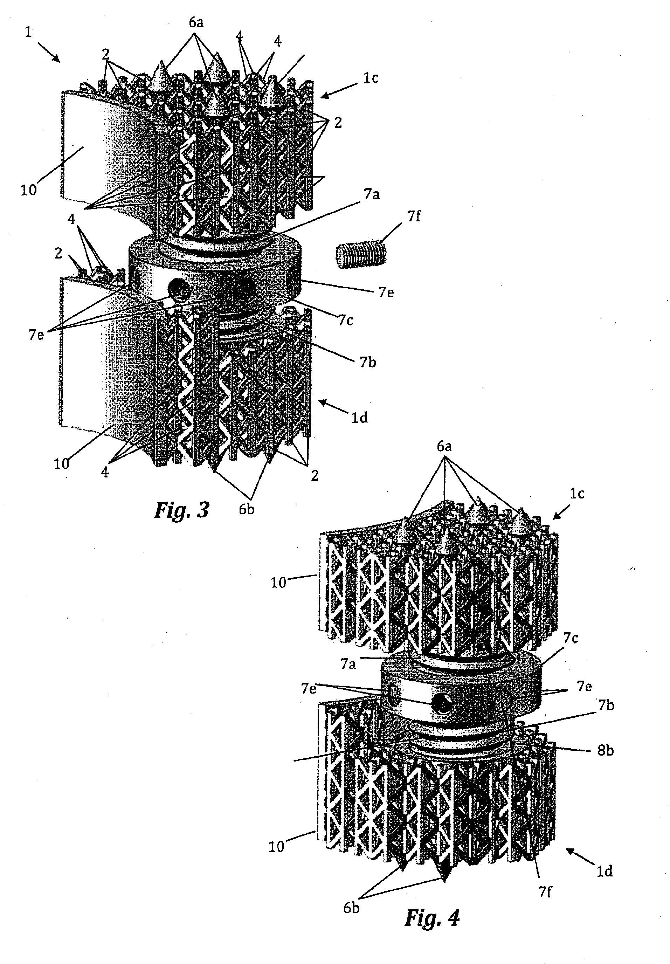

Figures 3 to 11 show an embodiment of the implant expansible in height by means of a vertical expansion mechanism. According to this embodiment, the spacing body -1- comprises an upper body -1 c- and a lower body -1d- coupled to one another by the vertical expansion mechanism for adjusting the implant in height between two adjacent vertebral bodies.

The expansion mechanism comprises a screw -7- which is threaded in respective fixed housings -8a, 8b- in the upper body -1 c- and in the lower body -1 d-, for which purpose the screw -7- has an upper helical thread -7a- and a lower helical thread -7b- having opposite helical trajectories and a fixed outer flange -7c- located between said threads -7a, 7b-.

The screw -7- further comprises an inner axial passage -7d- through which it is guided in a sliding manner on a telescopic assembly formed by an upper tube -9a-fixed to the fixed housing -8a- of the upper body -1 c- and a lower cylinder -9b- fixed to the fixed housing -8b-of the lower body -1d- which extend in the inner axial passage -7d- of the screw -7-.

The upper cylinder -9a- is a tube having a keyway -9c- in the form of an axial recess in its wall, whereas the lower cylinder -9b- has, protruding radially from its wall, a key -9d- in the form of an outer axial rib complementary to the mentioned axial recess. Since the tube -9a- is vertically slidable on the lower cylinder -9b-, the telescopic assembly formed by the keyway -9c- and the key -8d- makes the rotation between both cylinders impossible. On the other hand, since the fixed cylinders -9a, 9b- are located in the upper body -1c- and the lower body -1d-, respectively, the relative rotation between said bodies -1c, 1d- is also made impossible.

When a rotating movement is applied to the outer flange -7c-, the screw -7-rotates and, depending on the direction of the rotation, it expands or reduces the height of the implant to adapt the height of the implant to the site existing between the two adjacent vertebral bodies, which can be performed *in situ* with the implant already introduced between said vertebral bodies. To apply the rotating movement, the outer flange -7c- comprises radial threaded passages -7e- communicated with the inner axial passage -7d- in which the tip of a suitable instrument can be introduced. On the other hand, the threaded passages -7e- also serve for screwing a set-screw -7f- in at least one of the passages until it presses against the wall of the upper tube -9a-, and therefore the rotation of the screw -7- is blocked (see Figure 4). The set-screw -7f- comprises an outer thread -7g- and a slot -7h- at one of its bases serving for the application of a tightening tool, such as a screwdriver for example.

In the embodiment shown in Figures 1 and 2, the side of the spacing body -1-facing the spinal cord is provided with a concave wall -10- in the form of a plate with a smooth outer surface intended for preventing damage to the spinal cord. In the case of the embodiment shown in Figures 3 to 11, the concave wall -10- comprises two of these plates.

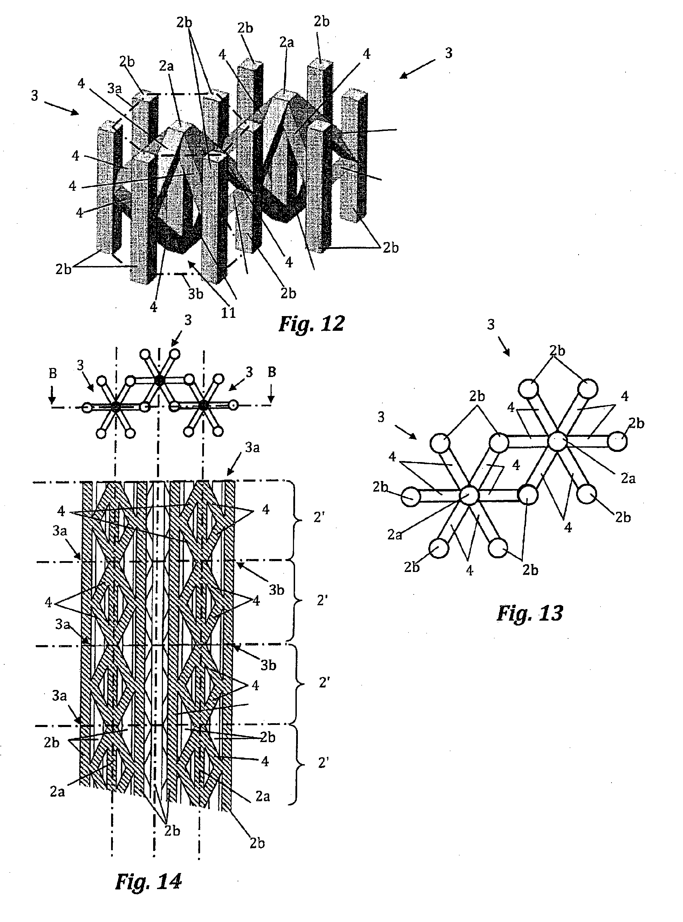

As can be seen in Figures 12 to 17, each unit cell -3- encloses an inner space -11- defined vertically between an upper base -3a- and a lower base -3b- of the unit cell -3-. Likewise, the inner space -11- of each unit cell -13- is laterally demarcated between respective vertical sections -2'- of at least three neighboring vertical bars -2a, 2b- arranged equidistantly from one another. Each vertical section -2'- is defined between the upper base -3a- and the lower base -3b- of the unit cell -3-.

On the other hand, each brace -4- of a unit cell connecting neighboring vertical bars -2- extends in an inclined manner between the upper base and the lower base of the unit cell such that the unit cells -3- form stacked strata of unit cells -3-.

As inferred from Figures 1 to 18, in the embodiments illustrated therein, each vertical bar -2a, 2b- in each unit cell -3- is attached to at least another neighboring vertical bar -2b, 2a- by means of a pair of inclined braces -4- with an inclination of 45°, with respective converging ends in a vertical section of a vertical bar -2b- in the intermediate part of the cell -3- and respective diverging ends attached to the other neighboring vertical bar -2a- in said upper part and in said lower part of the cell -3-, respectively.

In the embodiments illustrated in Figures 12 and 14, in each unit cell -3- the diverging ends of each pair of braces -4- are attached to a first vertical bar -2a-whereas the converging ends of the respective pairs of braces -4- are respectively attached to at least two second neighboring vertical bars -2b- that are close to one another. These second vertical bars -2b- are arranged radially with respect to the first vertical bar -2a-. Each brace -4- of each pair of braces -4- has a longitudinal axis intersecting with the first vertical bar -2a-. The longitudinal axes of the respective pairs of neighboring braces -4- intersect with the first vertical bar -2a- in equiangular arches of 60°.

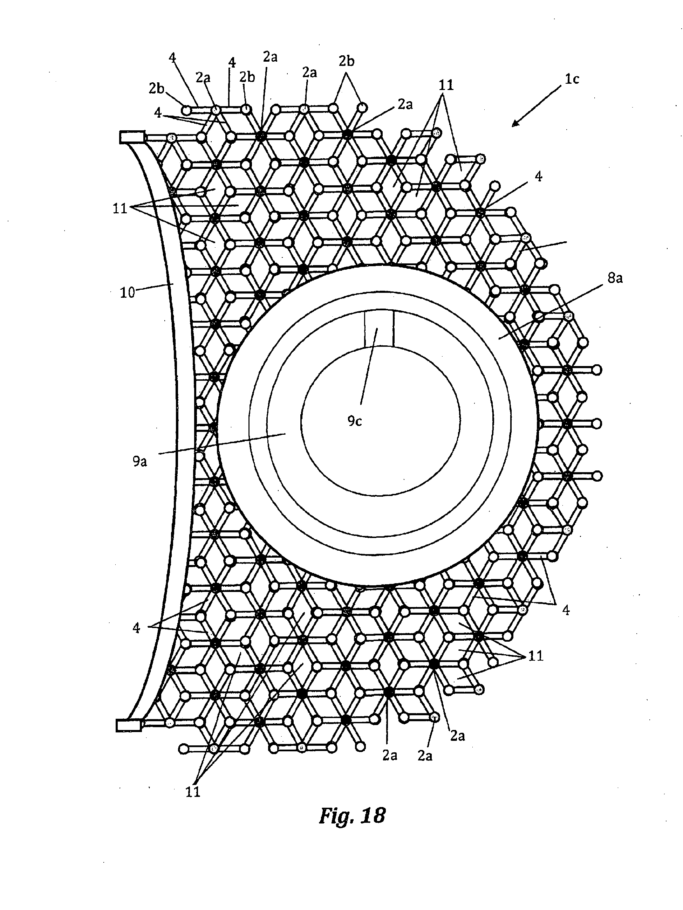

In Figure 18, in which the first vertical bars -2a- are depicted as solid circles, an embodiment of the upper body -1 c- of the spacing body of the implant made from unit cells such as those shown in Figures 12 to 17 can be seen in more detail. The upper body -1 c- integrates the housing -8a- and the upper tube -9a-. As can be seen, the vertical bars -2a, 2b- and the attachment braces -4- form the reticular structure defined above in the present description.

Figures 19 to 25 show other embodiments of the unit cells -3- from which the reticular structure of the spacing body -1- of the implant according to the present invention can be completely or partially made.

Figure 19 thus illustrates an embodiment of a unit cell -3- the inner space -11-of which is laterally demarcated by four vertical sections -2'- attached to one another by means of respective pairs of inclined inner braces -4- with converging ends centrally attached to one another in the inner space -11-. The diverging ends of the two braces -4- forming each pair of braces -4- are attached to one of the vertical sections -2'- in said upper part and in said lower part of the cell -3-, respectively.

Figure 20 illustrates an embodiment of a unit cell -3- the inner space -11- of which is also laterally demarcated by four vertical sections -2'-. The vertical sections -2-- are attached to one another by means of respective pairs of inclined radial inner braces -4- with converging ends centrally attached to one another in the inner space -11-. Likewise, the diverging ends of the two braces -4- forming each pair of braces -4- are attached to one of the vertical sections -2'- in said upper part and in said lower part of the cell -3-, respectively. According to this embodiment, the neighboring vertical sections -2'- are further attached by respective pairs of outer braces -4- the respective converging ends of which are also attached to one another, and the upper diverging ends of which are attached to neighboring vertical sections -2'- in the area of the upper base -3a- of the unit cell -3-, whereas the lower diverging ends thereof are attached to neighboring vertical sections -2'- in the area of the lower base -3b- of the unit cell -3-.

In the embodiment illustrated in Figure 21, the inner space -11- is also laterally demarcated between four vertical sections -2'-. Each vertical bar -2'- is attached to its neighboring vertical section -2- by means of a pair of inclined inner braces -4- with respective converging ends in an intermediate part of a vertical section -2'- and respective diverging ends attached to the other neighboring vertical section -2- in the area of the upper part -3a- and in the lower part -3b- of the cell -3-, respectively. On the other hand, the vertical sections -2'- are attached to one another by means of inclined radial inner braces -4-. Specifically, an upper assembly of radial braces -4- and a lower assembly of radial braces -4- can be seen. The upper assembly of braces -4- comprises respective pairs of inclined radial inner braces -4-with converging ends centrally attached to one another in the inner space -11- at a height defined between the upper base -3a- and the intermediate part of the cell -3-, whereas the diverging ends of the two braces -4- forming each pair of braces -4- are attached to one of the vertical sections -2'- in the area of the upper base -3a- and in said intermediate part of the cell -3, respectively. On the other hand, the lower assembly of braces -4- comprises respective pairs of inclined radial inner braces -4-with converging ends centrally attached to one another in the inner space -11- at a height defined between the intermediate part and the lower base of the cell -3-, whereas the diverging ends of the two braces -4- forming each pair of braces -4- are attached to one of the vertical sections -2'- in the intermediate part and the lower base of the cell -3-, respectively.

Figure 22 illustrates the embodiment of a unit cell -3- in which the inner space -11- is laterally demarcated by six angled bars -2d- with respective upper converging ends in the upper base -3a- of the unit cell -3- and respective lower converging ends in the lower base -3-of the cell -3-.

In the embodiment of the unit cell -3- shown in Figure 23, the inner space -11-is also laterally demarcated by six angled bars -2d- with respective upper converging ends in the upper base -3a- of the unit cell -3- and respective lower converging ends in the lower base -3- of the unit cell -3-. The upper converging ends and the lower converging ends of the angled bars are attached to opposite ends of a vertical section -2'- of a vertical bar (2) of the columns of unit cells (3) of the reticular structure.

Finally, in the embodiments illustrated in Figures 24 and 25, the respective vertical connecting elements connecting the respective upper and lower bases -3a, 3b- of the unit cell -3- are vertical connecting walls -2c- provided with through holes -2e- which in the case of Figure 24 are rectangular and in the case of Figure 25 circular.

## Claims

1. Osseointegrable implant for cervical corpectomy intended for replacing a damaged vertebral body between two vertebral bodies adjacent to the damaged vertebral body comprising a cylindrical spacing body (1) susceptible to housing graft material therein, and with an upper end (1 a) and a lower end (1 b), a plurality of side holes (5) communicated with the inside of the spacing body for introducing graft material, upper anchoring means (6a) protruding from the upper end (1a) of the spacing body (1) for anchoring the implant to an upper adjacent vertebral body, and lower anchoring means (6b) protruding from the lower end (1 b) of the spacing body (1) for anchoring the implant to a lower adjacent vertebral body, wherein
the spacing body (1) comprises a reticular supporting structure comprising a plurality of laterally interconnected columns of three-dimensional unit cells (3) attached to one another; each unit cell (3) encloses an inner space (11) defined vertically between an upper base (3a) and a lower base (3b) of the unit cell (3);
the inner spaces (11) of the unit cells (3) are laterally demarcated between vertical connecting elements (2, 2a, 2b, 2c, 2d) connecting the respective upper and lower bases (3a, 3b) of the cells (3);
**characterized in that**
the columns of unit cells (3) of the reticular structure are formed by a plurality of vertical bars (2, 2a, 2b) attached to one another by means of braces (4) extending between neighboring vertical bars (2, 2a, 2b);
the inner space (11) of each unit cell (3) is laterally demarcated between respective vertical sections (2') of at least three neighboring vertical bars (2, 2a, 2b);
each vertical section (2, 2') is defined between the upper base (3a) and the lower base (3b) of the unit cell (3), and each brace (4) of a unit cell (3) connecting neighboring vertical bars (2, 2a, 2b) extends between the upper base (3a) and the lower base (3b) of the unit cell (3) such that the unit cells (3) form stacked strata of unit cells (3).

2. Implant according to claim 1, **characterized in that** the neighboring vertical bars (2, 2a, 2b) are arranged equidistantly from one another.

3. Implant according to claim 1 or 2, **characterized in that** the braces (4) extend horizontally between neighboring vertical bars (2, 2a, 2b).

4. Implant according to claim 1 or 2, **characterized in that** the braces (4) extend in an inclined manner between neighboring vertical bars (2, 2a, 2b).

5. Implant according to claim 4, **characterized in that** the braces (4) have an inclination of 45°± 5° with respect to the vertical bars (2, 2a, 2b) to which they are attached.

6. Implant according to claim 4 or 5, **characterized in that** in at least some of the unit cells (3), each vertical bar (2, 2a, 2b) is attached to at least another neighboring vertical bar (2, 2a, 2b) by means of a pair of inclined braces (4) with respective converging ends in the vertical section (2') of a vertical bar (2, 2a, 2b) in an intermediate part of the unit cell (3), and respective diverging ends attached to the other neighboring vertical bar (2, 2a, 2b) in said upper part (3a) and in said lower part (3b) of the unit cell (3), respectively.

7. Implant according to claim 6, **characterized in that** in at least some of the unit cells (3)
the diverging ends of each pair of braces (4) are attached to a first vertical bar (2a);
the first vertical bar (2a) is attached to the diverging ends of at least two pairs of braces (4) the converging ends of which are attached respectively to at least two second neighboring vertical bars (2b) that are close to one another;
the second vertical bars (2b) are arranged radially with respect to the first vertical bar (2a);
the neighboring braces (4) attaching the second neighboring vertical bars (2b) with the first vertical bar (2a) have respective longitudinal axes intersecting with the first vertical bar (2a), defining respective equiangular arches with respect to one another;
the inner space (11) of each unit cell (3) is defined between the first vertical bar (2a) and the second vertical bars (2b).

8. Implant according to claim 7, **characterized in that** the longitudinal axes of said neighboring braces (4) define equiangular arches of 15° to 90° with respect to one another.

9. Implant according to claim 8, **characterized in that** the longitudinal axes of said neighboring braces (4) define equiangular arches of 60° with respect to one another.

10. Implant according to claim 1, **characterized in that** the respective vertical connecting elements connecting the respective upper and lower bases (3a, 3b) of at least one of the cells (3) are vertical connecting walls (2c) provided with through holes (2e).

11. Implant according to claim 1, **characterized in that** the respective vertical connecting elements connecting the respective upper and lower bases (3a, 3b) of at least some of the cells (3) are angled vertical connecting bars (2d) with respective upper converging ends in the upper base (3a) of the unit cell (3) and respective lower converging ends in the lower base (3b) of the unit cell (3).

12. Implant according to any one of the preceding claims, **characterized in that** the spacing body (1) comprises at least one concave wall (10).

13. Implant according to any one of the preceding claims, **characterized in that**
the spacing body (1) comprises an upper body (1c) and a lower body (1d) coupled to one another by a vertical expansion mechanism for adjusting the implant in height between the two adjacent vertebral bodies,
the expansion mechanism comprises a screw (7) which is threaded in respective fixed housings (8a, 8b) in the upper body (1c) and in the lower body (1d);
the screw (7) has an upper helical thread (7a) and a lower helical thread (7b) having opposite helical trajectories and a fixed outer flange (7c) located between said threads (7a, 7b);

14. Implant according to claim 13, **characterized in that** the screw has an inner axial passage (7d) through which it is guided in a sliding manner on a telescopic assembly formed by an upper cylinder (9a) fixed to the fixed housing (8a) of the upper body (1c) and a lower cylinder (9b) fixed to the fixed housing (8b) of the lower body (1d) which extend through the inner axial passage (7d) of the screw (7), at least one of said cylinders (9a, 9b) being a tubular cylinder (9a) which slides vertically on the other cylinder.

## Patentansprüche

1. Osseointegrierbares Implantat zur zervikalen Korporektomie, das zum Ersatz eines geschädigten Wirbelkörpers zwischen zwei zum geschädigten Wirbelkörper benachbarten Wirbelkörpern bestimmt ist, mit einem zylindrischen Abstandskörper (1), in dem Transplantatmaterial untergebracht sein kann, und mit einem oberen Ende (1a) und einem unteren Ende (1b), mehreren mit dem Inneren des Abstandskörpers kommunizierenden Seitenlöchern (5) zum Einbringen von Transplantatmaterial, einer oberen Verankerungseinrichtung (6a), die vom oberen Ende (1a) des Abstandskörpers (1) vorsteht, zum Verankern des Implantats an einem oberen benachbarten Wirbelkörper und einer unteren Verankerungseinrichtung (6b), die vom unteren Ende (1b) des Abstandskörpers (1) vorsteht, zum Verankern des Implantats an einem unteren benachbarten Wirbelkörper, wobei
der Abstandskörper (1) eine retikuläre Stützstruktur mit mehreren lateral miteinander verbundenen Säulen dreidimensionaler Einheitszellen (3) aufweist, die aneinander befestigt sind; jede Einheitszelle (3) einen Innenraum (11) umschließt, der zwischen einer oberen Basis (3a) und einer unteren Basis (3b) der Einheitszelle (3) vertikal definiert ist;
die Innenräume (11) der Einheitszellen (3) zwischen vertikalen Verbindungselementen (2, 2a, 2b, 2c, 2d) lateral abgegrenzt sind, die die jeweiligen oberen und unteren Basen (3a, 3b) der Zellen (3) verbinden;
**dadurch gekennzeichnet, dass**
die Säulen von Einheitszellen (3) der retikulären Struktur durch mehrere vertikale Stäbe (2, 2a, 2b) gebildet sind, die mit Hilfe von Streben (4) aneinander befestigt sind, die sich zwischen vertikalen Nachbarstäben (2, 2a, 2b) erstrecken;
der Innenraum (11) jeder Einheitszelle (3) zwischen jeweiligen vertikalen Teilstücken (2') mindestens dreier vertikaler Nachbarstäbe (2, 2a, 2b) lateral abgegrenzt ist;
jedes vertikale Teilstück (2, 2') zwischen der oberen Basis (3a) und der unteren Basis (3b) der Einheitszelle (3) definiert ist und sich jede Strebe (4) einer Einheitszelle (3), die vertikale Nachbarstäbe (2, 2a, 2b) verbindet, zwischen der oberen Basis (3a) und der unteren Basis (3b) der Einheitszelle (3) so erstreckt, dass die Einheitszellen (3) gestapelte Schichten aus Einheitszellen (3) bilden.

2. Implantat nach Anspruch 1, **dadurch gekennzeichnet, dass** die vertikalen Nachbarstäbe (2, 2a, 2b) in gleichem Abstand voneinander angeordnet sind.

3. Implantat nach Anspruch 1 oder 2, **dadurch gekennzeichnet, dass** sich die Streben (4) zwischen vertikalen Nachbarstäben (2, 2a, 2b) horizontal erstrecken.

4. Implantat nach Anspruch 1 oder 2, **dadurch gekennzeichnet, dass** sich die Streben (4) zwischen vertikalen Nachbarstäben (2, 2a, 2b) auf geneigte Weise erstrecken.

5. Implantat nach Anspruch 4, **dadurch gekennzeichnet, dass** die Streben (4) eine Neigung von 45° ± 5° im Hinblick auf die vertikalen Stäbe (2, 2a, 2b) haben, an denen sie befestigt sind.

6. Implantat nach Anspruch 4 oder 5, **dadurch gekennzeichnet, dass** in mindestens einigen der Einheitszellen (3) jeder vertikale Stab (2, 2a, 2b) an mindestens einem weiteren vertikalen Nachbarstab (2, 2a, 2b) mit Hilfe eines Paars von geneigten Streben (4) mit jeweiligen konvergierenden Enden im vertikalen Teilstück (2') eines vertikalen Stabs (2, 2a, 2b) in einem Zwischenteil der Einheitszelle (3) und jeweiligen divergierenden Enden befestigt ist, die am anderen vertikalen Nachbarstab (2, 2a, 2b) im Oberteil (3a) bzw. im Unterteil (3b) der Einheitszelle (3) befestigt sind.

7. Implantat nach Anspruch 6, **dadurch gekennzeichnet, dass** in mindestens einigen der Einheitszellen (3)
die divergierenden Enden jedes Paars von Streben (4) an einem ersten vertikalen Stab (2a) befestigt sind;
der erste vertikale Stab (2a) an den divergierenden Enden mindestens zweier Paare von Streben (4) befestigt ist, deren konvergierende Enden jeweils an mindestens zwei zweiten vertikalen Nachbarstäben (2b) befestigt sind, die nahe aneinander liegen;
die zweiten vertikalen Stäbe (2b) im Hinblick auf den ersten vertikalen Stab (2a) radial angeordnet sind;
die Nachbarstreben (4), die die zweiten vertikalen Nachbarstäbe (2b) am ersten vertikalen Stab (2a) befestigen, jeweilige Längsachsen haben, die den ersten vertikalen Stab (2a) schneiden, was jeweilige gleichwinklige Bögen zueinander definiert;
der Innenraum (11) jeder Einheitszelle (3) zwischen dem ersten vertikalen Stab (2a) und den zweiten vertikalen Stäben (2b) definiert ist.

8. Implantat nach Anspruch 7, **dadurch gekennzeichnet, dass** die Längsachsen der Nachbarstreben (4) gleichwinklige Bögen von 15° bis 90° zueinander definieren.

9. Implantat nach Anspruch 8, **dadurch gekennzeichnet, dass** die Längsachsen der Nachbarstreben (4) gleichwinklige Bögen von 60° zueinander definieren.

10. Implantat nach Anspruch 1, **dadurch gekennzeichnet, dass** die jeweiligen vertikalen Verbindungselemente, die die jeweilige obere und untere Basis (3a, 3b) mindestens einer der Zellen (3) verbinden, vertikale Verbindungswände (2c) sind, die mit Durchgangslöchern (2e) versehen sind.

11. Implantat nach Anspruch 1, **dadurch gekennzeichnet, dass** die jeweiligen vertikalen Verbindungselemente, die die jeweiligen oberen und unteren Basen (3a, 3b) mindestens einiger der Zellen (3) verbinden, abgewinkelte vertikale Verbindungsstäbe (2d) mit jeweiligen oberen konvergierenden Enden in der oberen Basis (3a) der Einheitszelle (3) und jeweiligen unteren konvergierenden Enden in der unteren Basis (3b) der Einheitszelle (3) sind.

12. Implantat nach einem der vorstehenden Ansprüche, **dadurch gekennzeichnet, dass** der Abstandskörper (1) mindestens eine konkave Wand (10) aufweist.

13. Implantat nach einem der vorstehenden Ansprüche, **dadurch gekennzeichnet, dass** der Abstandskörper (1) einen oberen Körper (1c) und einen unteren Körper (1d) aufweist, die durch einen vertikalen Expansionsmechanismus zum Einstellen des Implantats in seiner Höhe zwischen den beiden benachbarten Wirbelkörpern miteinander gekoppelt sind,
der Expansionsmechanismus eine Schraube (7) aufweist, die in jeweiligen festen Gehäusen (8a, 8b) im oberen Körper (1c) und im unteren Körper (1d) eingeschraubt ist;
die Schraube (7) ein oberes Spiralgewinde (7a) und ein unteres Spiralgewinde (7b) mit entgegengesetzten Spiralbahnen und einen festen Außenflansch (7c) hat, der zwischen den Gewinden (7a, 7b) liegt.

14. Implantat nach Anspruch 13, **dadurch gekennzeichnet, dass** die Schraube einen Innenaxialdurchgang (7d) hat, durch den sie auf einer Teleskopanordnung gleitend geführt wird, die durch einen am festen Gehäuse (8a) des oberen Körpers (1c) fixierten oberen Zylinder (9a) und einen am festen Gehäuse (8b) des unteren Körpers (1d) fixierten unteren Zylinder (9b) gebildet ist, die sich durch den Innenaxialdurchgang (7d) der Schraube (7) erstrecken, wobei mindestens einer der Zylinder (9a, 9b) ein Röhrenzylinder (9a) ist, der auf dem anderen Zylinder vertikal gleitet.

## Revendications

1. Implant osséo-intégrable pour corpectomie des cervicales destiné à remplacer un corps vertébral endommagé entre deux corps vertébraux adjacents au corps vertébral endommagé, comprenant un corps d'espacement cylindrique (1) susceptible d'abriter un matériau de greffe et comportant une extrémité supérieure (1a) et une extrémité inférieure (1 b), une pluralité de trous latéraux (5) en communication avec l'intérieur du corps d'espacement pour introduire un matériau de greffe, des moyens d'ancrage supérieurs (6a) faisant saillie de l'extrémité supérieure (1 a) du corps d'espacement (1) pour ancrer l'implant sur un corps vertébral supérieur adjacent, et des moyens d'ancrage inférieurs (6b) faisant saillie de l'extrémité inférieure (1 b) du corps d'espacement (1) pour ancrer l'implant sur un corps vertébral inférieur adjacent, dans lequel :
le corps d'espacement (1) comprend une structure de support réticulaire comprenant une pluralité de colonnes latéralement interconnectées de cellules unitaires tridimensionnelles (3) fixées l'une à l'autre; chaque cellule unitaire (3) enserre un espace interne (11) défini verticalement entre une base supérieure (3a) et une base inférieure (3b) de la cellule unitaire (3) ;
les espaces internes (11) des cellules unitaires (3) sont démarqués latéralement entre des éléments de raccordement verticaux (2, 2a, 2b, 2c, 2d) raccordant les bases supérieures et inférieures respectives (3a, 3b) des cellules (3) ;
**caractérisé en ce que** :
les colonnes des cellules unitaires (3) de la structure réticulaire sont formées par une pluralité de barres verticales (2, 2a, 2b) fixées l'une à l'autre au moyen d'attelles (4) s'étendant entre des barres verticales voisines (2, 2a, 2b) ;
l'espace interne (11) de chaque cellule unitaire (3) est latéralement démarquée entre des sections verticales respectives (2') d'au moins trois barres verticales voisines (2, 2a, 2b) ;
chaque section verticale (2, 2') est définie entre la base supérieure (3a) et la base inférieure (3b) de la cellule unitaire (3) et chaque attelle (4) d'une cellule unitaire (3) raccordant des barres verticales voisines (2, 2a, 2b) s'étend entre la base supérieure (3a) et la base inférieure (3b) de la cellule unitaire (3) de sorte que les cellules unitaires (3) forment des couches empilées de cellules unitaires (3).

2. Implant selon la revendication 1, **caractérisé en ce que** les barres verticales voisines (2, 2a, 2b) sont aménagées mutuellement à équidistance.

3. Implant selon la revendication 1 ou la revendication 2, **caractérisé en ce que** les attelles (4) s'étendent horizontalement entre des barres verticales voisines (2, 2a, 2b).

4. Implant selon la revendication 1 ou la revendication 2, **caractérisé en ce que** les attelles (4) s'étendent de manière inclinée entre des barres verticales voisines (2, 2a, 2b).

5. Implant selon la revendication 4, **caractérisé en ce que** les attelles (4) ont une inclinaison de 45°±5° par rapport aux barres verticales (2, 2a, 2b) auxquelles elles sont fixées.

6. Implant selon la revendication 4 ou la revendication 5, **caractérisé en ce que**, dans au moins certaines des cellules unitaires (3), chaque barre verticale (2, 2a, 2b) est fixée à au moins une autre barre verticale voisine (2, 2a, 2b) au moyen d'une paire d'attelles inclinées (4) avec des extrémités convergentes respectives de la section verticale (2') d'une barre verticale (2, 2a, 2b) dans une partie intermédiaire de la cellule unitaire (3) et des extrémités divergentes respectives fixées à l'autre barre verticale voisine (2, 2a, 2b) dans ladite partie supérieure (3a) et dans ladite partie inférieure (3b) de la cellule unitaire (3), respectivement.

7. Implant selon la revendication 6, **caractérisé en ce que**, dans au moins certaines des cellules unitaires (3),
les extrémités divergentes de chaque paire d'attelles (4) sont fixées à une première barre verticale (2a) ;
la première barre verticale (2a) est fixée aux extrémités divergentes d'au moins deux paires d'attelles (4), dont les extrémités convergentes sont fixées respectivement à au moins deux secondes barres verticales voisines (2b) qui sont proches l'une de l'autre ;
les secondes barres verticales (2b) sont aménagées radialement par rapport à la première barre verticale (2a) ;
les attelles voisines (4) fixant les secondes barres verticales voisines (2b) à la première barre verticale (2a) ont des axes longitudinaux respectifs coupant la première barre verticale (2a), définissant mutuellement des arcs équiangulaires respectifs ;
l'espace interne (11) de chaque cellule unitaire (3) est défini entre la première barre verticale (2a) et les secondes barres verticales (2b).

8. Implant selon la revendication 7, **caractérisé en ce que** les axes longitudinaux desdites attelles voisines (4) définissent des arcs équiangulaires mutuels de 15° à 90°.

9. Implant selon la revendication 8, **caractérisé en ce que** les axes longitudinaux desdites attelles voisines (4) définissent des arcs équiangulaires mutuels de 60°.

10. Implant selon la revendication 1, **caractérisé en ce que** les éléments de raccordements verticaux respectifs raccordant les bases supérieure et inférieure respectives (3a, 3b) d'au moins l'une des cellules (3) sont des parois de raccordement verticales (2c) pourvues de trous traversants (2e).

11. Implant selon la revendication 1, **caractérisé en ce que** les éléments de raccordements verticaux respectifs raccordant les bases supérieures et inférieures respectives (3a, 3b) d'au moins certaines des cellules (3) sont des barres de raccordement verticales coudées (2d) avec des extrémités convergentes supérieures respectives dans la base supérieure (3a) de la cellule unitaire (3) et des extrémités convergentes inférieures respectives dans la base inférieure (3b) de la cellule unitaire (3).

12. Implant selon l'une quelconque des revendications précédentes, **caractérisé en ce que** le corps d'espacement (1) comprend au moins une paroi concave (10).

13. Implant selon l'une quelconque des revendications précédentes, **caractérisé en ce que** :
le corps d'espacement (1) comprend un corps supérieur (1c) et un corps inférieur (1d) couplés l'un à l'autre par un mécanisme d'expansion verticale pour ajuster l'implant en hauteur entre les deux corps vertébraux adjacents,
le mécanisme d'expansion comprend une vis (7) qui est vissée dans des boîtiers fixes respectifs (8a, 8b) dans le corps supérieur (1 c) et dans le corps inférieur (1d) ;
la vis (7) a un filet hélicoïdal supérieur (7a) et un filet hélicoïdal inférieur (7b) ayant des trajectoires hélicoïdales opposées et un collier externe fixe (7c) situé entre lesdits filets (7a, 7b).

14. Implant selon la revendication 13, **caractérisé en ce que** la vis a un passage axial interne (7d) à travers lequel elle est guidée en mode coulissant sur un ensemble télescopique formé par un cylindre supérieur (9a) fixé au boîtier fixe (8a) du corps supérieur (1 c) et un cylindre inférieur (9b) fixé au boîtier fixe (8b) du corps inférieur (1d) qui s'étend à travers le passage axial interne (7d) de la vis (7), au moins l'un desdits cylindres (9a, 9b) étant un cylindre tubulaire (9a) qui coulisse verticalement sur l'autre cylindre.
